(19) Europäisches Patentamt
European Patent Office
Office européen des brevets

(11) Veröffentlichungsnummer: **0 236 667 A2**

(12) # EUROPÄISCHE PATENTANMELDUNG

(21) Anmeldenummer: **87100111.1**

(51) Int. Cl.4: **A61K 31/12**

(22) Anmeldetag: **08.01.87**

(30) Priorität: **16.01.86 DE 3601065**

(43) Veröffentlichungstag der Anmeldung: **16.09.87 Patentblatt 87/38**

(84) Benannte Vertragsstaaten: **AT BE CH DE ES FR GB IT LI LU NL SE**

(71) Anmelder: **BEHRINGWERKE Aktiengesellschaft**
**Postfach 1140**
**D-3550 Marburg 1(DE)**

(72) Erfinder: **Kraemer, Hans Peter, Dr.**
**Birkenweg 16**
**D-3550 Marburg(DE)**
Erfinder: **Krohn, Karsten, Prof. Dr.**
**Am Turmsberg 49**
**D-3300 Braunschweig(DE)**

(74) Vertreter: **Meyer-Dulheuer, Karl-Hermann, Dr. et al**
**HOECHST Aktiengesellschaft Zentrale Patentabteilung Postfach 80 03 20**
**D-6230 Frankfurt/Main 80(DE)**

(54) **Chinon-Derivate als Antitumormittel.**

(57) Es wird beschrieben, daß die Verbindungen der Formel I

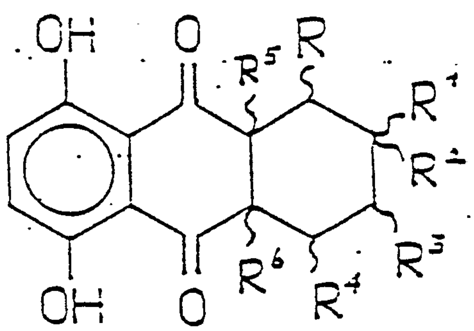

I

worin
R H, OH oder $OSi(CH_3)_3$,
$R^1$ H oder Alkyl,
$R^2$ OH oder Alkoxy und
$R^3$ OH oder $R^2$ und $R^3$ gemeinsam O,
$R^4$ H oder Alkoxy oder $R^3$ und $R^4$ gemeinsam O und
$R^5$ und $R^6$ H oder zusammen O bedeuten, als Antitumormittel verwendet werden können.

EP 0 236 667 A2

## Chinon-Derivate als Antitumormittel

Die Erfindung betrifft die Verwendung von Chinon-Derivaten der Formel I

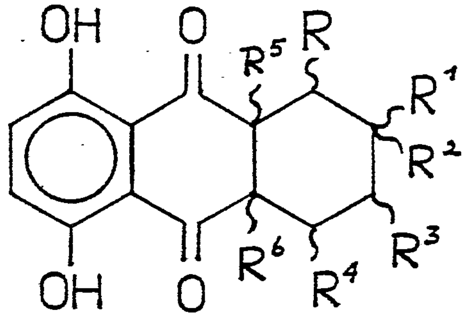

worin
R H, OH oder $OSi(CH_3)_3$,
$R^1$ H oder Alkyl,
$R^2$ OH oder Alkoxy und
$R^3$ OH oder $R^2$ und $R^3$ gemeinsam O,
$R^4$ H oder Alkoxy oder $R^3$ und $R^4$ gemeinsam O und
$R^5$ und $R^6$ H oder zusammen O bedeuten,
als Antitumormittel.

Dabei können die Substituenten in alpha-oder beta-Position stehen.

Die Alkyl-oder Alkoxygruppen enthalten bevorzugt 1-4 Kohlenstoffatome.

Bevorzugte Verbindungen sind solche, bei denen eine oder mehrere der folgenden Bedingungen zutrifft:
R Hydroxy oder $OSi(CH_3)_3$;
$R^1$ Methyl;
$R^2$ und $R^3$ OH oder zusammen O;
$R^4$ H.

Die Erfindung betrifft weiterhin Arzneimittel, welche eine Verbindung der Formel I enthalten, insbesondere die im Folgenden als Verbindungen 1, 2, 3, 4 und 5 bezeichneten.

Verbindungen der Formel I und ihre Herstellung ist beschrieben in J.Chem.Res. 9, 306-307 (1978) und Ann. Heft 7, 1311-1328 (1985).

Nachweis der antitumoralen Wirkung:

Die antitumorale Wirkung der Verbindungen der Formel I kann in verschiedenen Testsystemen wie im Proliferationstest oder an Leukämiezellen der Maus festgestellt werden.

Koloniebildung von L1210-Leukämiezellen in Soft Agar:
Diese Methode dient zum Nachweis eines Einflußes der Testsubstanzen auf das Wachstumsverhalten der Zellen über mehrere Generationen (bei einer Zellzykluszeit von 10-12 Stunden werden in der Testzeit von 7 Tagen ca. 14 aufeinander folgende Generationen beobachtet). Zytostatisch wirksame Substanzen bewirken in diesem Test eine Reduktion der zu beobachtenden Koloniezahl gegenüber einer unbehandelten Kontrolle. Im einzelnen wurde der Test wie folgt durchgeführt:
500 Leukämiezellen pro Platte wurden mit unterschiedlichen Konzentrationen der Testsubstanz 1 Stunde bei 37°C inkubiert. Anschließend wurden die Zellen zweimal mit McCoy-5A Medium gewaschen und - schließlich in Petrischalen nach Zusatz von 0,3% Agar ausgegossen. Kontrollen wurden lediglich mit frischem Medium inkubiert. Anstelle der einstündigen Inkubation wurden in manchen Fällen unterschiedliche Konzentrationen der Testsubstanz der oberen Agarschicht zugemischt, um so eine kontinuierliche Exposition der Zellen über die gesamte Inkubationszeit zu erreichen. Nach Erstarren des Agars wurden die Platten im Brutschrank 7 Tage bei 37°C inkubiert (5% $CO_2$, 95% relative Luftfeuchtigkeit). Anschließend wurde die Zahl der entstandenen Kolonien mit einem Durchmesser ab 60μ gezählt. Die Ergebnisse wurden ausgedrückt als der Quotient der Koloniezahl in behandelten Agarplatten und der Koloniezahl in der unbehandelten Kontrolle. Aus der so erhaltenen Dosis-Wirkungskurve wurde die $IC_{50}$ als Maß für die Wirksamkeit der Testsubstanz ermittelt. Als Vergleichssubstanzen dienten bei diesem Test die alkylierenden Zytostatika Diacetyldianhydrogalactitol (Diac DAG) und Melphalan, sowie das interkalierende Zytostatikum Adriamycin. Die Ergebnisse sind in Tabelle 1 zusammengefaßt.

Tabelle 1

| Substanz | Stammzellassay cont. µg/ml | Stammzellassay 1 h µg/ml |
|---|---|---|
| Diac DAG | 0.5 | 2.5 |
| Melphalan | 0.6 | 0.9 |
| Adriamycin | 0.02 | 0.04 |
| Verbindung 1 | 0.02 | 0.07 |
| Verbindung 2 | 0.02 | 0.016 |
| Verbindung 3 | 0.024 | 0.014 |
| Verbindung 4 | 0.03 | 0.03 |
| Verbindung 5 | 0.05 | 0.05 |

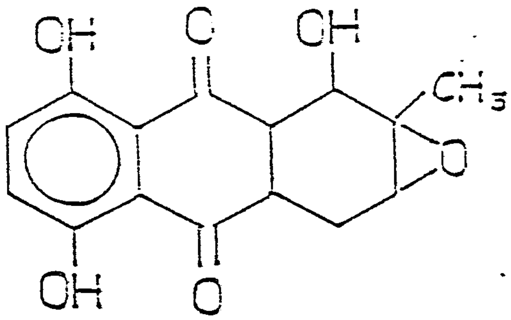

Verbindung 1

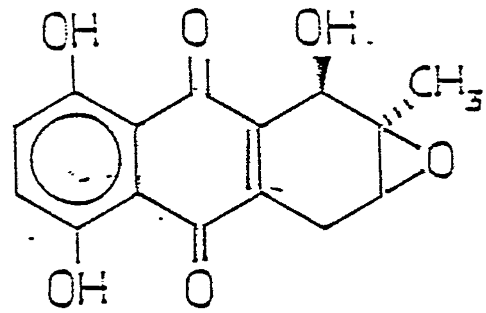

Verbindung 2

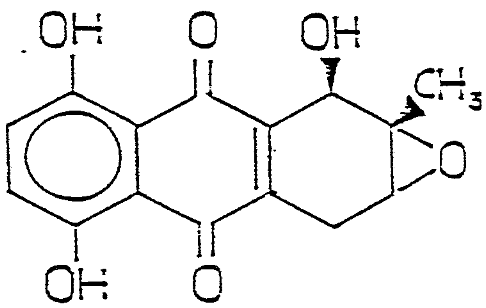

Verbindung 3

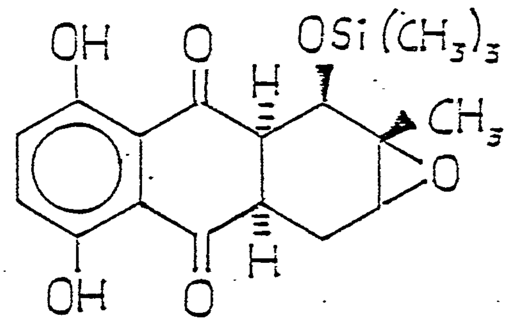

Verbindung 4

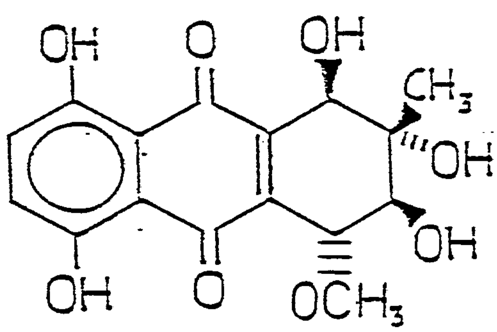

Verbindung 5

Tabelle 1 zeigt, daß die erfindungsgemäßen Verbindungen überraschenderweise unter in vitro-Bedingungen an L1210-Leukämiezellen der Maus eine für alkylierende Zytostatika unerwartet hohe Zytotoxizität aufweisen. Diese Zytotoxizität entspricht in den allermeisten Fällen derjenigen von Anthracyclin -Antibiotika, die struktuell mit den hier untersuchten Substanzen nicht vergleichbar sind. Weiterhin ist überraschend, daß durch das Öffnen des Epoxidringes zum entsprechenden Diol eine ebenfalls hoch zytotoxisch wirksame Verbindung entsteht, obwohl die alkylierenden Eigenschaften des Moleküls durch diese Ringöffnung beseitigt werden (Verbindung 5).

Die Verbindungen der Formel I werden in einer Dosis von 0,1 bis 300 mg pro kg Körpergewicht angewandt, beispielsweise gelöst in physiologischer Kochsalzlösung.

## Ansprüche

1. Verwendung einer Verbindung der Formel I

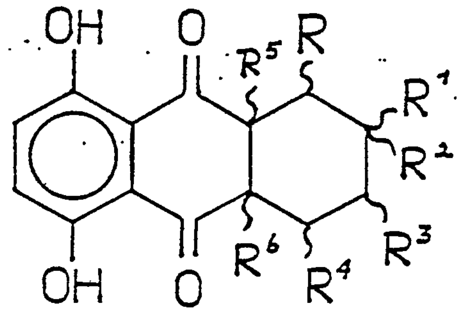

worin
R H, OH oder $OSi(CH_3)_3$,
$R^1$ H oder Alkyl,
$R^2$ OH oder Alkoxy und
$R^3$ OH oder $R^2$ und $R^3$ gemeinsam O,
$R^4$ H oder Alkoxy oder $R^3$ und $R^4$ gemeinsam O und
$R^5$ und $R^6$ H oder zusammen O bedeuten,
als Antitumormittel.

2. Verwendung einer Verbindung nach Anspruch 1, dadurch gekennzeichnet, daß R OH, $R^1$ Alkyl, $R^2$ OH oder $R^2$ und $R^3$ gemeinsam O, $R^4$ H oder $R^3$ und $R^4$ gemeinsam O und $R^5$ und $R^6$ H sind.

3. Verwendung einer Verbindung der Formel I in Anspruch 1 in einer Dosis von 0,1 bis 300 mg per kg Körpergewicht als Antitumormittel.

4. Verwendung einer Verbindung der Formel I in Anspruch 1 in einem Verfahren zur Herstellung eines Antitumormittels.

Patentansprüche für folgenden Vertragsstaat : ES

1. Verwendung einer Verbindung der Formel I

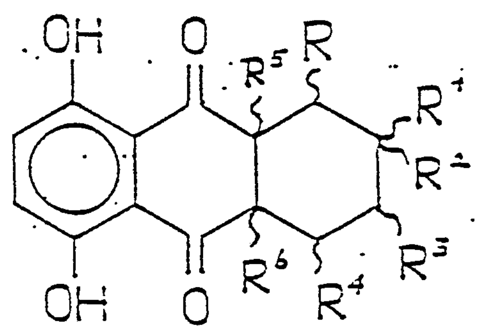

I

worin
R H, OH oder $OSi(CH_3)_3$,
$R^1$ H oder Alkyl,
$R^2$ OH oder Alkoxy und
$R^3$ OH oder $R^2$ und $R^3$ gemeinsam O,

$R^4$ H oder Alkoxy oder $R^3$ und $R^4$ gemeinsam O und $R^5$ und $R^6$ H oder zusammen O bedeuten, in einem Verfahren zur Herstellung eines Antitumormittels.

2. Verwendung einer Verbindung nach Anspruch 1, dadurch gekennzeichnet, daß R OH, $R^1$ Alkyl, $R^2$ OH oder $R^2$ und $R^3$ gemeinsam O, $R^4$ H oder $R^3$ und $R^4$ gemeinsam O und $R^5$ und $R^6$ H sind.

3. Verwendung einer Verbindung nach Anspruch 1, dadurch gekennzeichnet, daß das Antitumormittel pro Dosis (75 kg Körpergewicht) 0,1 bis 300 mg dieser Verbindung enthält.